# EUROPEAN PATENT SPECIFICATION

(11) **EP 1 406 999 B1**
(45) Date of publication and mention of the grant of the patent: **02.12.2009**
(21) Application number: 02752865.2
(22) Date of filing: 04.07.2002
(51) Int. Cl.: C12N 1/02

(54) **RECOVERY OF BIOLEACHING MICROBES**
EXTRAKTION VON MIKROBEN DURCH MIKROBIELLE LAUGUNG
RECUPERATION DE MICROBES DE BIOLESSIVAGE

(30) Priority: 16.07.2001 ZA 200105817
(43) Date of publication of application: 14.04.2004
(73) Proprietor: BHP Billiton SA Limited, Johannesburg, 2001 (ZA)
(72) Inventor: DU PLESSIS, Chris, Andre, c/o BHP Billiton, 2125 Randburg (ZA)
(74) Representative: Wood, Graham
(86) International application number: PCT/ZA2002/000110
(87) International publication number: WO 2003/008565

(56) References cited:
- WO-A-02/42504
- HARRISON, S.T.L. ET AL: "The use of mini-hydrocyclones for differential separations within mineral slurries subjected to bioleaching" MINERALS ENGINEERING (1997), 10(5), 529-535 , XP001117795

## Description

### BACKGROUND OF THE INVENTION

This invention relates generally to bioleaching and more particularly is concerned with a method for recovering bioleaching microbes.

Bioleaching is the term used to refer to the microbial oxidation of reduced iron or sulphide, contained in solid mineral particles, with the subsequent liberation of valuable metals associated with these particles. Continuous culture bioleaching reactors currently in operation, commercially as well as at pilot plant scale, of which the applicant is aware, operate without separation of hydraulic retention time from solids retention time. This implies that, under certain conditions, microbial growth rates are the limiting factor in the bioleaching process. Under such conditions process benefits could be achieved by recovering microbial cells from the effluent bioleach liquor and returning these cells to the aerated bioleach reactors. The net effect of such a supplementation of recovered microbes, in high concentrations and low volumes to the bioleaching reactors, would be that the microbial accumulation of cells in these reactors would exceed that which could be achieved by microbial growth rate alone.

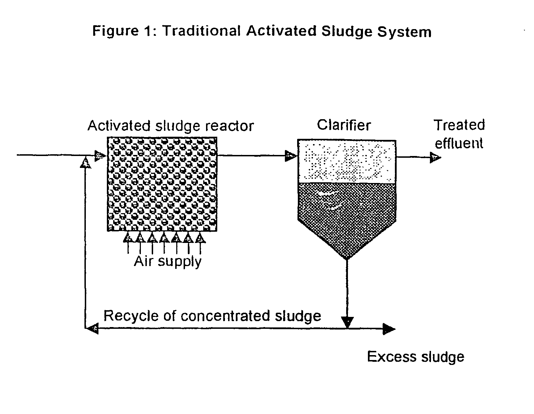

The activated sludge process, which is illustrated schematically in Figure 1, was developed to address water pollution problems due to a dense population and advanced industry. During wastewater treatment, organic compounds in the water are biodegraded in an aerated reactor, mainly by bacteria, resulting in the build-up of a bacterial biomass in the reactor. In order to maintain the biomass in the aerated reactor, the inflow rate of fluid into the reactor should not exceed the rate at which the bacteria proliferate. Such a scenario would result in the washout of bacteria from the reactor with an associated loss of biodegradation capacity for the incoming organic compounds. The throughput of influent into such a reactor is therefore limited by the growth rate of the microbes in the reactor. This limitation can be overcome, however, by separating the bacterial biomass (sludge) from the rest of the liquid. This step is typically performed in a clarifier. The relatively higher density of the sludge allows for gravitational settling with an overflow of treated effluent. The concentrated sludge can then be returned to the aerated sludge reactor. This process allows for the accumulation of sludge in the aerated reactor and is referred to as activated sludge. The net effect of the process is that biomass, produced in the process of biodegrading the organic compounds contained in the influent, is not lost from the system, apart from a small amount that is purged to limit the accumulation of excess sludge. This results in a high concentration of biomass in the activated sludge reactor, which allows for a high liquid input flow rate and treatment efficiency of the reactor.

Microbial recovery of cells from bioleach bioractors is significantly more complex than the operation of a typical activated sludge plant used for wastewater treatment. In the case of wastewater treatment two phases exist, viz the biomass sludge phase (biological solids phase) and the liquid phase. In the case of bioleaching reactors three distinguishable phases exist, viz the mineral solids particles (mineral solids), the microbial cell biomass (biological solids phase) and the liquid phase. In addition, the microbial solids phase is not as uniform as in the case of activated sludge plants. In the bioleaching scenario, biological solids are either attached onto the mineral particle surfaces or freely suspended. Separation of the biomass phase from the other phases poses significant technical difficulties and challenges. These factors are a prohibitive barrier to the implementation of cell recycling, as in the activated sludge process, to bioleaching technology.

Schiraldi et al (Reference 1) describe a microfiltration bioreactor which achieves a high cell density in *Sulfolobus solfataricus* fermentation. Use is made of a hollow fibre microfiltration unit with the main aim being to improve yield in the growth medium by removal of toxic compounds in the growth medium. Cell concentration occurs in situ in the growth reactor and there is no mention of cell recycling as in the activated sludge-type system. The medium does not contain solid mineral particles, nor is iron contained in any significant amount in the medium. The medium contains yeast extract and other organic compounds and has a pH in the range of from 3.5 to 3.8. The microfiltration unit is applied to a heterotrophic application.

The techniques described by Schiraldi et al are not useful for recovering microbial cells from bioleaching reactors. It should be borne in mind that the bioleaching environment is unique and displays the following characteristics:
(a) extreme pH conditions of 1-2;
(b) highly corrosive environments caused by the presence of high concentrations of oxidised ferric and other metals;
(c) high concentrations of ferric sulphate precipitates such as jarosite;
(d) a three phase system containing liquids (with salts in solution), biological solids phase (microbial biomass), and a minerals solids phase (precipitates, oxidised mineral particles and non-oxidised mineral particles);
(e) the exclusive presence of autotrophic microbes that do not require an organic carbon source;
(f) high temperatures (as high as 80°C) in the case of some bioleaching reactions;
(g) the fact that microbial flocculation and aggregation do not readily occur in bioleaching reactors; and
(h) the potential presence of suspended mineral solids and precipitates, together with microbial cells, in the feed solution.

### SUMMARY OF THE INVENTION

The invention provides a method of recovering microbial cells in a bioleaching process which includes the steps of subjecting a slurry produced in a bioleaching plant to a solid/liquid separation process, and extracting microbial cells from the resulting liquid.

The microbial cells may be separated from metal in solution, in the liquid.

The microbial cells may be extracted by subjecting the liquid to a centrifugal process which may be carried out continuously, or on a batch basis.

Preferably the microbial cells are extracted by subjecting the liquid to a membrane filtration process.

When use is made of the membrane filtration process the cells may be recovered by continuous concentration of the cells or the cells may be accumulated onto an inner surface of the membrane and are then removed in any appropriate way for example by back flushing or washing.

The microbial cells may be extracted using a plurality of extraction phases which are operated in series.

The bioleaching plant may include at least one bioleaching reactor, and typically includes a plurality of bioleaching reactors connected in series.

The method may extend to at least one of the following steps:
(a) recycling the microbial cells to the said at least one bioleaching reactor to increase the rate of microbial cell accumulation and assist in increased bioleaching efficiency;
(b) storage and packaging of the recovered cells, including freeze-drying and cryo-preservation for future use or as a backup inoculum;
(c) inoculation of a new bioleaching reactor or re-inoculation of a currently used bioleaching reactor which may be under-performing; and
(d) extraction of bioproducts such as enzymes and proteins from the microbial biomass i.e. the extracted cells.

### BRIEF DESCRIPTION OF THE DRAWINGS

The invention is further described by way of examples with reference to the accompanying drawings in which:
Figure 1 illustrates an activated sludge system (prior art) which has already been described in the preamble to this specification,
Figure 2 is a block diagram representation of a method of recovering bioleaching microbes in accordance with the principles of the invention,
Figure 3 is a more detailed illustration of the process shown in Figure 2,
Figure 4 is a diagram of a ceramic membrane microfilter for use in the methods shown in Figures 2 and 3, and
Figure 5 schematically depicts a centrifugal separation system which can be used in place of a ceramic membrane microfilter, in the method of the invention, to recover bioleaching microbes.

### DESCRIPTION OF PREFERRED EMBODIMENTS

Figure 1 depicts a typical activated sludge process and has already been referred to in the preamble to this specification.

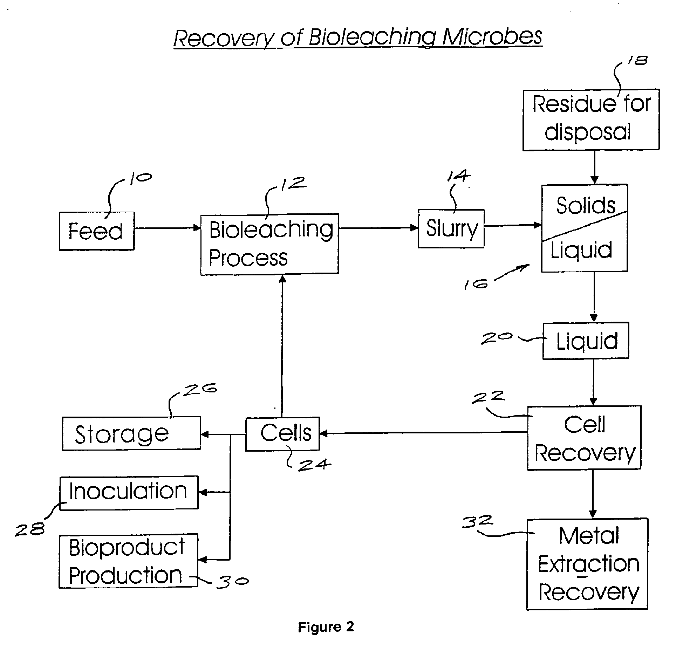

Figure 2 of the accompanying drawings is a flow chart representation of the method of the invention for recovering bioleaching microbial cells making use of a microfiltration membrane.

Bioleaching may be described as a process in which the biooxidation of ferrous iron and sulphides occurs in continuous culture agitated and aerated reactors, with the subsequent release of metals such as copper into solution². In the case of gold and other precious metals, the metals of interest do not go into solution but are biobeneficiated by the fact that the sulphides are oxidised, resulting in lower cyanide consumptions upon extraction of such metals from the remaining residue. This process can take place in a single reactor or a train of several reactors in series.

Referring to Figure 2 a concentrate feed 10 which contains a desired metal such as gold or copper, which is to be recovered, is fed to a bioleaching process 12. A slurry 14, produced by the bioleaching process, is fed to a solid/liquid separation stage 16. The oxidised minerals solids residue 18 from the separation stage 16 is treated for disposal.

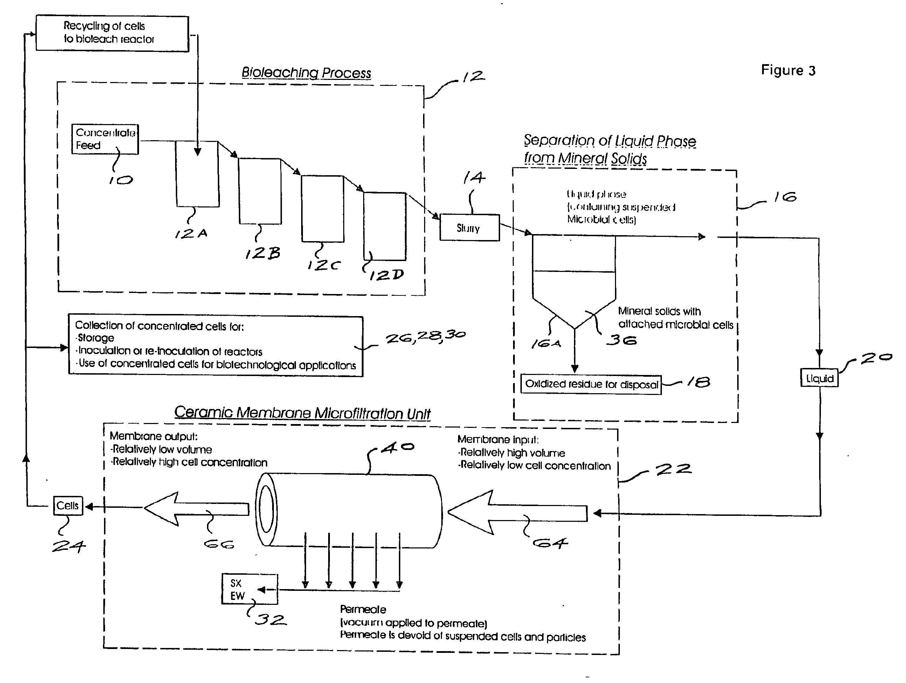

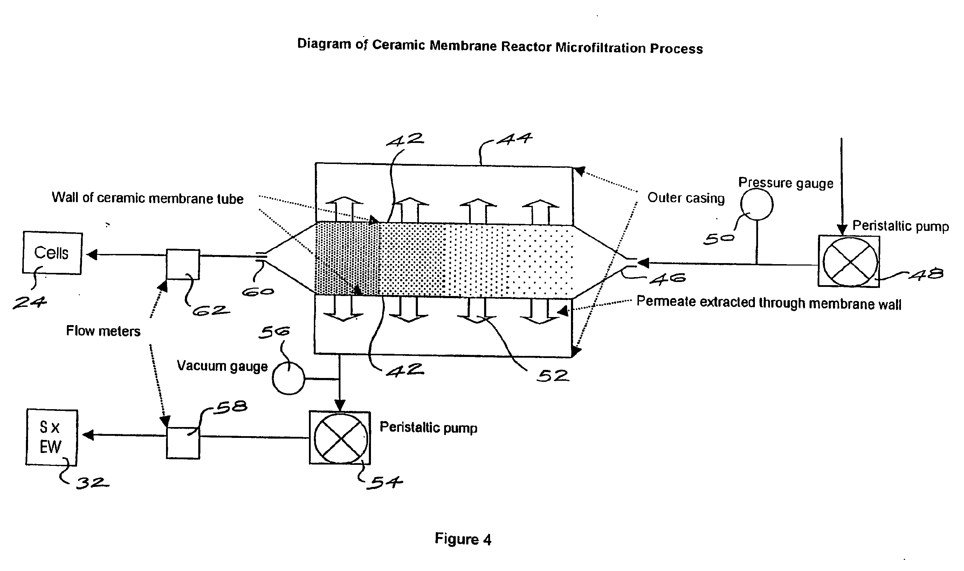

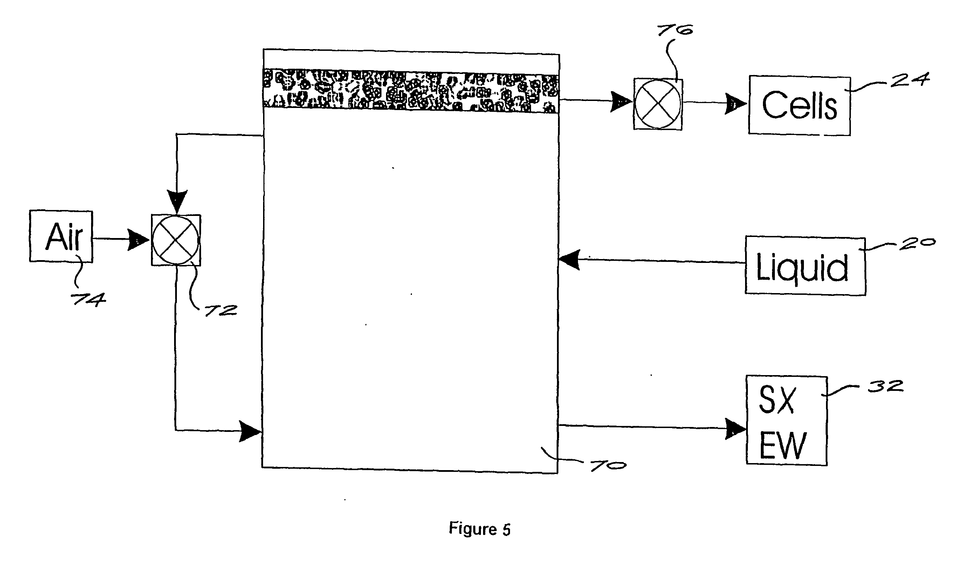

The liquid or supernatant 20 from the process 16 is subjected to a cell recovery step 22 which may be effected by making use of a centrifugal technique (see Figure 5), or by making use of a membrane filtration process (see Figures 3 and 4), which separates the suspended cells 24 from the liquid 20. The recovered cells may be recycled to the process 12, be stored (block 26), be used for inoculation purposes (block 28), or be used for bioproduct production (block 30).

The metal remaining in the liquid from which the cells 24 are extracted is then recovered using a suitable process 32 which is known in the art e.g. solvent extraction or electrowinning.

Figure 3 is a more detailed depiction of the method of the invention and, where applicable, like reference numerals are used to designate like steps or components.

Referring to Figure 3 the concentrate feed 10 is fed to a bioleaching plant 12 which includes a succession of bioleaching reactors designated 12A, 12B, 12C, etc. which are connected in series. This type of process is known in the art. The slurry effluent 14 from the bioleaching chain of reactors reports to a solids/liquid separation process 16 which typically is based on the use of a clarifier or thickener 16A. During this process the minerals solids particles, together with the microbial cells which are attached to these particles, settle out from suspension under gravitation forces. A flocculent agent may be added to assist in such separation. The supernatant 20 then contains a suspension of remaining (non-attached) microbial cells. The oxidised minerals solids residue 36 from this process is treated for disposal (step 18). The retention time in the solids/liquid separation system should be sufficiently short to ensure ongoing viability of the microbial cells. A variation of a gravitational solids/liquid separation step would be the use of a hydrocyclone or any similar process in which the gravitational selectivity and separation is induced or takes advantage of the relative density differences between the microbial cells and the minerals solids particles.

The supernatant 20 from the solids/liquid separation process, containing suspended microbial cells, is treated by using a membrane filtration process 22 to separate the suspended cells from the solution. This may be effected in various ways. In a first instance, shown in Figure 3, the liquid 20 is passed into a ceramic microfiltration membrane 40, which is shown in further detail in Figure 4. The membrane has a cylindrical wall 42 and is mounted inside a casing 44. The liquid 20 is pumped into an inlet 46 of the cell by means of a peristaltic pump 48, or any similar device. The pressure of the system, which is a measure of the permeability of the membrane, is monitored by means of a gauge 50.

Permeate 52 passes through the membrane wall and is collected inside the housing. The permeate flow through the membrane wall is assisted by means of a vacuum induced inside the housing, around the membrane, by means of a peristaltic pump 54 or any similar device. The level of the vacuum inside the housing is monitored by means of a gauge 56.

The permeate 52 contains metals in solution and is directed to a known metal recovery process 32, as has been described in connection with Figure 2. A flow meter 58 is used to provide a measure of the permeate flow rate from the casing 44.

The cells 24 exit the membrane tube via an outlet 60 and are continuously recovered. The rate at which the cells leave the membrane tube is measured by means of a flow meter 62.

On the input side of the membrane, i.e. at the inlet 46, the flow rate is relatively high with a relatively low cell concentration. This situation is designated in Figure 3 by means of a relatively large arrowhead 64. On the other hand at the outlet 60 the flow rate is relatively low but the cell concentration is relatively high, as is indicated by means of a relatively small arrowhead 66.

Regular membrane maintenance procedures are put in place in order to ensure sustainable efficient cross membrane flow.

Several membrane units may be used in series to improve efficiency and in order to allow for sequential and automated back-flushing and membrane maintenance procedures to be implemented. In the case of thermophilic microbial cells, the membrane reactor, the solids/liquid separation system, and the solution entering and exiting the membrane reactor should preferably be maintained at a temperature sufficiently high to maintain metabolic activity of these cells.

As has been described in connection with Figures 2 and 3 the recovered concentrated cell suspension 24 could be used for any, or a combination, of the following:
(a) recycled to primary or secondary bioleaching reactors in order to increase the rate of microbial cell accumulation and assist in increased bioleaching efficiency;
(b) storage and packaging, including freeze drying and cryo-preservation for future use or as backup inoculum;
(c) inoculation of new reactors or re-inoculation of currently used reactors that may be under performing; and
(d) extraction of enzymes, proteins or other bioproducts from microbial biomass.

The process of the invention could also be applied in a system where a step is introduced to remove attached microbial cells from solid mineral particles.

The following bioleaching process benefits can be achieved directly or indirectly from the recovery and recycling of cells to, and from, a bioleaching reactor plant:
(a) increased cell concentrations in the reactors;
(b) higher minerals oxidation rates as a result of increased cell numbers;
(c) control of the microbial concentration density in different reactors by selective addition to different reactors;
(d) increased process robustness. The higher cell concentrations would be less effected by process upsets and inhibitory conditions than would be the case for reactors with lower cell concentrations;
(e) any microbial adaptations which occur in the reactors would be retained and recycled. This would result in the eventual proliferation of any such mutated or adapted organism if such an adaptation provided the particular cell with a competitive advantage under the prevailing reactor conditions; and
(f) cells with various growth rate kinetics could be accommodated in the reactors. In continuous culture reactor systems where the hydraulic retention time is identical to the solids retention time and the biological sludge retention time, the microbial growth rate is controlled by the hydraulic retention time. In such a reactor, at steady state, the microbial growth rate of the cells is equal to the dilution rate (reciprocal of the hydraulic retention time). Microbes with a maximum specific growth rate less than the prevailing dilution rate would, therefore, be accommodated in such a reactor. Microbes with a maximum specific growth rate greater than the prevailing dilution rate would, however, be lost (washed out) from the reactor even if such microbes have superior affinity for ferrous and/or sulphide (smaller half saturation constants for these substrates) or have other superior qualities beneficial in the bioleaching process. Discrimination in the continuous culture process is, therefore, mainly based on growth rates at the prevailing conditions. By recovering and recycling the cells to the reactors, the microbial cell (biological sludge) retention time is separated from the hydraulic and mineral solids retention times. This allows for the accommodation of microbes with a greater range of maximum specific growth rates and, therefore, also of microbes with other beneficial bioleaching properties.

Effective monitoring of the membrane recovery system could include the following elements:
(a) monitoring of viability (activity) of microbial cells by determining their activity prior to and after membrane recovery using respirometry (Micro-Oxymax) methods (oxygen and carbon dioxide consumption) or ferrous oxidation capacity;
(b) monitoring of microbial cell numbers by direct microscopic counting, particle size analyses (Cell Facts) and protein concentration determinations;
(c) monitoring of the cross-membrane flux by measuring the balance of flow rates of the influent, effluent and permeates respectively; and
(d) monitoring of the cross-membrane pressure drops by measuring the inlet pressure and vacuum pressure applied to obtain a specific flow rate across the membrane.

### Example

The process described hereinbefore has been tested by using equipment with the following specifications:
- Membrane type: Ceramic microfiltration membrane
- Length: 0.212 m
- Diameter: 0.012 m
- Module setup: 3 membranes in series
- Membrane area: 0.024 m² per module
- Membrane wall thickness: 0.7 mm

### Summary of Results

The system was operated using two regimes: vacuum assisted permeate removal and constant flux operation. Both operating regimes reached and exceeded a 50-fold concentration factor in a single pass objective, at a flux of 56 m⁻² h⁻¹ and 99% biomass retention. This far exceeds the performance of commercial systems, in which a 20-fold concentration is typically achieved at fluxes of 20 - 40 m⁻² h⁻¹.

Figure 5 schematically illustrates a centrifugal based system which can be used in place of the membrane microfilter shown in Figures 3 and 4.

The supernatant 20 is directed into a centrifuge 70 which is spun at high speed and which separates the microbial cells from the remaining solution in the centrifuge. The cells 24 are drawn from the centrifuge and are used in any of the applications which have been described. Solution which is depleted of cells is treated (step 32) for metal recovery e.g. by means of solvent extraction or electrowinning.

The centrifugal separation system can be operated on a batch or continuous basis to achieve results which are similar to those obtained by means of the membrane filter.

### References

1. Schiraldi C, Marulli F, Di Lemaia I, Martino A, De Rosa M. 1999. A microfiltration bioreactor to achieve high cell density in Sulfolobus solfataricus fermentation. Extremophiles 3, 199-204.
2. MIRO, The use of micro-organisms in the minerals and metals industries, Technical Review Series No 6., Vol 3, 1995-2000. Minerals Industry Research Organisation, Lichfield UK. ISBN 1 872440 22 3.

## Claims

1. A method of recovering microbial cells in a bioleaching process which includes the steps of subjecting a slurry produced in a bioleaching plant to a solid/liquid separation process, and extracting microbial cell from the resulting liquid, **characterised in that** the microbial cells are extracted using one of the following techniques:
a) a centrifugal process which is carried our continuously or on a batch basis; or
b) by continuously concentrating the cells by subjecting the liquid to a membrane filtration process wherein the cells are accumulated onto an inner surface of the membrane and are then removed by back flushing or washing.

2. A method according to claim 1 wherein the microbial cells are separated from metal in solution, in the liquid.

3. A method according to any one of claims 1 to 2 wherein the bioleaching plant includes a plurality of bioleaching reactors connected in series.

4. A method according to any one of claims 1 to 3 which includes the step of recycling the microbial cells to at least one bioleaching reactor to increase the rate of microbial cell accumulation and assist in increased bioleaching efficiency.

5. A method according to any one of claims 1 to 4 which includes the step of storage and packaging of the recovered cells, including freeze-drying and cryo-preservation for future use or as a backup inoculum.

6. A method according to any one of claims 1 to 5 which includes the step of inoculation of a new bioleaching reactor or re-inoculation of a currently used bioleaching reactor which may be under-performing.

7. A method according to any one of claims 1 to 6 which includes the step of extraction of bioproducts such as enzymes and proteins from the extracted cells.

## Patentansprüche

1. Verfahren zum Gewinnen von mikrobiellen Zellen in einem Bioleaching-Prozess, das die folgenden Schritte beinhaltet: Unterziehen eines in einer Bioleaching-Anlage produzierten Schlamms einem Fest/Flüssig-Trennverfahren und Extrahieren von mikrobiellen Zellen aus der resultierenden Flüssigkeit, **dadurch gekennzeichnet, dass** die mikrobiellen Zellen mit einer der folgenden Methoden extrahiert werden:
a) mit einem Schleuderverfahren, das kontinuierlich oder diskontinuierlich ausgeführt wird; oder
b) durch kontinuierliches Konzentrieren der Zellen, indem die Flüssigkeit einem Membranfiltrationsverfahren unterzogen wird, wobei sich die Zellen auf einer Innenseite der Membran ansammeln und dann durch Rückspülung oder -wäsche entfernt werden.

2. Verfahren nach Anspruch 1, wobei die mikrobiellen Zellen von Metall in Lösung in der Flüssigkeit getrennt werden.

3. Verfahren nach einem der Ansprüche 1 bis 2, wobei die Bioleaching-Anlage mehrere in Reihe geschaltete Bioleaching-Reaktoren beinhaltet.

4. Verfahren nach einem der Ansprüche 1 bis 3, das den Schritt des Rezirkulierens der mikrobiellen Zellen zu wenigstens einem Bioleaching-Reaktor beinhaltet, um die mikrobielle Zellansammlungsrate zu erhöhen und eine erhöhte Bioleaching-Leistung zu unterstützen.

5. Verfahren nach einem der Ansprüche 1 bis 4, das den Schritt des Aufbewahrens und Verpackens der gewonnenen Zellen beinhaltet, einschließlich einer Gefriertrocknung und Kryokonservierung für den zukünftigen Gebrauch oder als Reserve-Inokulum.

6. Verfahren nach einem der Ansprüche 1 bis 5, das den Schritt des Inokulierens eines neuen Bioleaching-Reaktors oder Reinokulierens eines derzeit gebrauchten Bioleaching-Reaktors, der möglicherweise eine zu geringe Leistung erbringt, beinhaltet.

7. Verfahren nach einem der Ansprüche 1 bis 6, das den Schritt des Extrahierens von Bioprodukten wie Enzyme und Proteine aus den extrahierten Zellen beinhaltet.

## Revendications

1. Procédé de récupération de cellules microbiennes dans le cadre d'un biolessivage, qui comprend les étapes consistant à soumettre une boue produite dans une installation de biolessivage à un procédé de séparation solides/liquides et à extraire du liquide résultant les cellules microbiennes, **caractérisé en ce que** les cellules microbiennes sont extraites au moyen de l'une des techniques suivantes :
a) procédé centrifuge exécuté de façon continue ou par lots ; ou
b) concentration continue des cellules en soumettant le liquide à un procédé de filtration par membrane durant lequel les cellules sont accumulées sur une surface intérieure de la membrane, puis éliminées par rinçage ou lavage à contre-courant.

2. Procédé selon la revendication 1, selon lequel les cellules microbiennes sont séparées du métal en solution dans le liquide.

3. Procédé selon la revendication 1 ou 2, selon lequel l'installation de biolessivage comprend une pluralité de réacteurs de biolessivage connectés en série.

4. Procédé selon l'une quelconque des revendications 1 à 3, qui comprend l'étape de recyclage des cellules microbiennes vers au moins un réacteur de biolessivage pour accroître le taux d'accumulation des cellules microbiennes et favoriser l'augmentation du rendement du biolessivage.

5. Procédé selon l'une quelconque des revendications 1 à 4, qui comprend l'étape de stockage et de conditionnement des cellules récupérées, y compris leur lyophilisation et leur cryoconservation pour usage ultérieur ou comme inoculum de réserve.

6. Procédé selon l'une quelconque des revendications 1 à 5, qui comprend l'étape d'inoculation d'un nouveau réacteur de biolessivage ou la réinoculation d'un réacteur de biolessivage en cours d'emploi mais dont les performances sont peut-être réduites.

7. Procédé selon l'une quelconque des revendications 1 à 6, qui comprend l'étape d'extraction de bioproduits tels qu'enzymes et protéines hors des cellules extraites.
